(19) Europäisches Patentamt
European Patent Office
Office européen des brevets

(11) Veröffentlichungsnummer: **0 041 923**
**B1**

(12) # EUROPÄISCHE PATENTSCHRIFT

(45) Veröffentlichungstag der Patentschrift: **07.03.84**

(51) Int. Cl.³: **C 07 C 101/18**, C 07 C 99/00

(21) Anmeldenummer: **81810217.0**

(22) Anmeldetag: **03.06.81**

(54) **Verfahren zur Herstellung von N-(1'-Alkoxycarbonyläthyl)-2,6-dialkylanilinen.**

(30) Priorität: **09.06.80 US 157762**

(43) Veröffentlichungstag der Anmeldung: **16.12.81 Patentblatt 81/50**

(45) Bekanntmachung des Hinweises auf die Patenterteilung: **07.03.84 Patentblatt 84/10**

(84) Benannte Vertragsstaaten: **AT BE CH DE FR GB IT LI NL SE**

(56) Entgegenhaltungen:
**DE - A - 2 812 169**
**US - A - 4 025 648**
**US - E - 29 468**

(73) Patentinhaber: **CIBA-GEIGY AG, Postfach, CH-4002 Basel (CH)**

(72) Erfinder: **Grieder, Alfred, Dr., Ob den Reben 15, CH-4461 Böckten (CH)**
Erfinder: **Coers, Klaus Jürgen, Lochackerstrasse 10, CH-4153 Reinach (CH)**
Erfinder: **Labuhn, Peter, Schwarzackerstrasse 57, CH-4303 Kaiseraugst (CH)**

**EP 0 041 923 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von N-(1'-Alkoxycarbonyläthyl)-2,6-dialkylanilinen

Die vorliegende Erfindung bezieht sich auf ein Verfahren zur Herstellung von N-(1'-Alkoxycarbonyläthyl)-2,6-dialkylanilinen der Formel I:

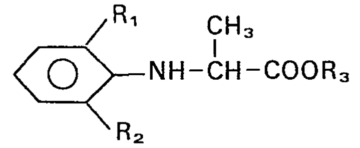

(I)

in welcher $R_1$ und $R_2$ je Methyl oder Äthyl bedeuten und $R_3$ für eine Alkylgruppe mit 1 bis 4 Kohlenstoffatomen steht, durch Umsetzung eines 2,6-Dialkylanilins der Formel II:

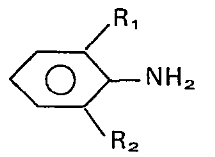

(II)

in welcher $R_1$ und $R_2$ die oben angegebene Bedeutung haben, mit einem 2-Halogenpropionsäureester der Formel III:

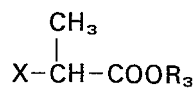

(III)

in welcher $R_3$ die oben angegebene Bedeutung hat und X für Chlor oder Brom steht.

Die N-(1'-Alkoxycarbonyläthyl)-2,6-dialkylaniline der Formel I sind wertvolle Zwischenprodukte für die Herstellung von pestizid wirksamen Verbindungen. Sie können beispielsweise durch Umsetzung mit Säurechloriden, wie Chloracetylchlorid, Methoxyacetylchlorid oder Furan-2-carbonsäurechlorid in entsprechende N-Acylaniline übergeführt werden, die sich durch eine hervorragende Wirkung gegen phytopathogene Mikroorganismen auszeichnen und daher ausgedehnte Anwendung im Pflanzenschutz finden. Solche N-Acylaniline sowie ihre Herstellung und Verwendung sind beispielsweise in den US-Patenten Nrn. 4008066, 4094990 und 4151299 beschrieben.

Unter den N-Acylanilinen der vorgenannten Art, die durch Acylierung der erfindungsgemäss herstellbaren N-(1'-Alkoxycarbonyläthyl)-2,6-dimethylaniline der Formel I hergestellt werden können, sind insbesondere das N-Methoxyacetyl-N-(1'-methoxycarbonyläthyl)-2,6-dimethylanilin und das N-(2''-Furoyl)-N-(1'-methoxycarbonyläthyl)-2,6-dimethylanilin zu nennen.

Die Herstellung von N-(1'-Alkoxycarbonyläthyl)-2,6-dialkylanilinen der Formel I durch Umsetzung von 2,6-Dialkylanilinen der Formel II mit α-Halogenpropionsäureestern der Formel III ist einerseits wegen der durch die orthoständigen Alkylgruppen bedingten sterischen Hinderung und andererseits wegen der Hydrolyseempfindlichkeit der α-Halogenpropionsäureester problematisch. Zur Lösung der mit der Durchführung dieses Verfahrens verbundenen Probleme wurden bereits verschiedene Vorschläge gemacht.

Es ist bekannt, N-Alkoxycarbonylmethyl-2,6-dialkylaniline durch Umsetzung von α-Halogenessigsäureestern und 2,6-Dialkylanilinen in Gegenwart von wässerigen Alkalimetallhydroxiden herzustellen. Nach dieser Methode werden die gewünschten Produkte infolge von Nebenreaktionen, wie N,N-Dialkylierung und Hydrolyse der Estergruppe, nur in ungenügender Reinheit und in unbefriedigenden Ausbeuten erhalten (US Patent Nr. 3882162).

Es wurde bereits vorgeschlagen, die Nachteile des vorgenannten Verfahrens dadurch zu vermeiden, dass man die Umsetzung des 2,6-Dialkylanilins mit dem α-Halogencarbonsäureester in Gegenwart von überschüssigem 2,6-Dialkylanilin als säurebindendes Mittel und in Gegenwart einer katalytischen Menge des Hydrochlorids des betreffenden 2,6-Dialkylanilins bei Temperaturen von 100 bis 250° C durchzuführen. Doch auch mit diesem Verfahren liegen die erreichbaren Ausbeuten unter 70% der Theorie (US Patent Nr. 3882162).

Ferner wurde bereits vorgeschlagen, kernsubstituierte Aniline bei Temperaturen von 100 bis 175° C in Gegenwart eines tertiären Amins als säurebindendes Mittel mit α-Halogencarbonsäureestern umzusetzen, wobei gemäss einer bevorzugten Ausführungsform dieses Verfahrens die Umsetzung in überschüssigem Ester als Lösungsmittel durchgeführt und zur Beschleunigung der Reaktion das tertiäre Amin bereits zu Beginn der Umsetzung in Form eines Salzes, beispielsweise in Form des Hydrochlorids, zugesetzt wird. Nach diesem Verfahren werden Umsätze an Anilin von 90 bis 96%, eine Selektivität von 78 bis 90% und Ausbeuten an N-(1'-Alkoxycarbonylalkyl)anilinen von 70 bis 86% d.Th. erreicht, wobei die Ausbeuteangaben nicht auf tatsächlich isoliertem Reinprodukt, sondern auf analytischer Bestimmung des Gehalts an Reinprodukt im Rohprodukt beruhen (CH Patent Nr. 572017).

Weiterhin ist aus der deutschen Offenlegungsschrift Nr. 2350944 bekannt, 2,6-Dimethylanilin bei 120 bis 125°C in Gegenwart von Natriumhydrogencarbonat als säurebindende Mittel mit dem dreifach molaren Überschuss an 2-Brompropionsäuremethylester umzusetzen. Bei diesem Verfahren wird N-(1'-Methoxycarbonyläthyl)-2,6-dimethylanilin in einer Ausbeute von 79,6% der Theorie erhalten.

Wie der vorstehende Überblick über den Stand der Technik zeigt, ist es mit den bisher bekannten Verfahren nicht möglich, N-(1'-Alkoxycarbonyläthyl)-2,6-dialkylaniline in befriedigender Ausbeute und Reinheit herzustellen. Es ist daher das Ziel der vorliegenden Erfindung, ein Verfahren vorzusehen, das die Nachteile der bekannten Verfahren vermeidet und das die Herstellung von N-(1'-Alkoxycarbonyläthyl)-2,6-dialkylanilinen der Formel I in zufriedenstellender Ausbeute und Reinheit auf einfache Weise ermöglicht.

Gemäss vorliegender Erfindung wird vorgeschlagen, dass man die Umsetzung des 2,6-Dialkylanilins der Formel II mit dem 2-Halogenpropionsäureester der Formel III bei 110 bis 130°C in Gegenwart eines Alkalijodids als Katalysator und eines Alkalicarbonats oder Alkalihydrogencarbonats als Säureakzeptor in überschüssigem 2,6-Dialkylanilin der Formel II als Lösungsmittel durchführt, während der Umsetzung das gebildete Wasser durch Destillation entfernt und die nach Zugabe von Wasser und Phasentrennung erhaltene organische Phase destillativ aufarbeitet.

Unter den 2-Halogenpropionsäureestern der Formel II sind die 2-Chlorpropionsäureester wegen ihres niedrigeren Preises bevorzugt. Besonders geeignet ist der 2-Chlorpropionsäuremethylester.

Das erfindungsgemäss im Überschuss zu verwendende 2,6-Dialkylanilin der Formel II wird vorteilhaft in Mengen von 1,5 bis 2,5 mol pro Mol 2-Halogenpropionsäureester der Formel III eingesetzt. Bei Verwendung von weniger als 1,5 mol 2,6-Dialkylanilin der Formel II pro Mol 2-Halogenpropionsäureester der Formel III sind Umsatz und Ausbeute niedriger. Die Verwendung von mehr als 2,5 mol 2,6-Dialkylanilin der Formel II pro Mol 2-Halogenpropionsäureester der Formel III ist grundsätzlich möglich. Die Anwendung derartig grosser Überschüsse ist jedoch im Hinblick auf den mit der destillativen Entfernung des überschüssigen 2,6-Dialkylanilins verbundenen Aufwand unwirtschaftlich. Vorzugsweise werden 1,6 bis 1,8 mol 2,6-Dialkylanilin der Formel II pro Mol 2-Halogenpropionsäureester der Formel III verwendet.

Als Alkalijodide kommen Lithium-, Natrium-, Kalium-, Cäsium- und Rubidiumjodid in Betracht. Bevorzugte Alkalijodide sind Natrium- umd Kaliumjodid. Das Alkalijodid wird in der Regel in einer Menge von 0,02 bis 0,3 mol pro Mol 2-Halogenpropionsäureester eingesetzt. Vorzugsweise verwendet man das Alkalijodid in einer Menge von 0,03 bis 0,15 mol pro Mol 2-Halogenpropionsäureester.

Als säurebindende Mittel sind erfindungsgemäss Alkali- und Alkalihydrogencarbonate, wie Lithium-, Natrium-, Kalium-, Rubidium- und Cäsiumcarbonat, sowie Lithiumhydrogen-, Natriumhydrogen-, Kaliumhydrogen-, Rubidiumhydrogen- und Cäsiumhydrogencarbonat geeignet. Bevorzugte säurebindende Mittel sind Natrium-, Kalium-, Natriumhydrogen- und Kaliumhydrogencarbonat. Die säurebindenden Mittel werden in der Regel in etwa stöchiometrischer Menge oder in einem Überschuss eingesetzt. Vorteilhaft werden die säurebindenden Mittel in einer Menge von 1 bis 3 Eq. bezogen auf den zu bindenden Halogenwasserstoff verwendet. Vorzugsweise verwendet man 1,1 bis 1,3 Eq. säurebindendes Mittel bezogen auf den zu bindenden Halogenwasserstoff.

Innerhalb des angegebenen Temperaturbereichs von 110 bis 130°C, in dem die Umsetzung eines 2,6-Dialkylanilins der Formel II mit einem 2-Halogenpropionsäureester der Formel III durchgeführt wird, sind Temperaturen von 120 bis 125°C bevorzugt. Das während der Umsetzung eines 2,6-Dialkylanilins der Formel II mit einem 2-Halogenpropionsäureester der Formel III in Gegenwart eines Alkalicarbonats bzw. Alkalihydrogencarbonats gebildete Wasser wird vorteilhaft während der Umsetzung durch azeotrope Destillation abgetrennt.

Nach beendigter Umsetzung wird das Reaktionsgemisch abgekühlt und durch Extraktion mit Wasser von Alkalisalzen befreit. Anschliessend wird aus der organischen Phase das überschüssige 2,6-Dialkylanilin der Formel II durch Destillation abgetrennt. Die destillative Abtrennung des 2,6-Dialkylanilins der Formel II erfolgt vorteilhaft im Vakuum. Das als Rückstand erhaltene N-(1'-Alkoxycarbonyläthyl)-2,6-dialkylanilin der Formel I kann in der Regel als solches für weitere Umsetzungen, beispielsweise für die eingangs genannte Umsetzung mit Carbonsäurechloriden, verwendet werden. Falls ein spezieller Anwendungszweck dies erforderlich macht, kann das Produkt jedoch auch durch eine Vakuumrektifikation gereinigt werden.

Gemäss einer bevorzugten Ausführungsform des erfindungsgemässen Verfahrens setzt man pro Mol 2-Halogenpropionsäureester der Formel III 1,7 mol 2,6-Dialkylanilin der Formel II ein und führt die Umsetzung bei 120 bis 125°C in Gegenwart von 0,03 bis 0,15 mol Natriumjodid pro Mol 2-Halogenpropionsäureester der Formel III und in Gegenwart von 0,55 bis 0,65 mol Natriumcarbonat pro Mol 2-Halogenpropionsäureester der Formel III durch. Nach dieser bevorzugten Ausführungsform lassen sich insbesondere 2,6-Dimethylanilin und 2-Chlorpropionsäuremethylester vorteilhaft umsetzen.

Mit dem erfindungsgemässen Verfahren wird es möglich, die N-(1'-Alkoxycarbonyläthyl)-2,6-dialkylaniline der Formel I in höheren Ausbeuten und in besserer Qualität herstellen als nach den bisher bekannten Verfahren. Das erfindungsgemässe Verfahren kann auf einfache Weise in üblichen Apparaturen in technischem Massstab durchgeführt werden und eignet sich insbesondere auch für die kontinuierliche Durchführung. Im Hinblick auf die hohe Ausbeute und Qualität der Verfahrensprodukte und den geringen apparativen Aufwand kann das erfindungsgemässe Verfahren auch als besonders wirtschaftlich angessehen werden.

Das erfindungsgemässe Verfahren bietet gegenüber bekannten Verfahren auch ökologische Vorteile, da infolge der hohen Selektivität und der hohen Ausbeute nur minimale Mengen an Neben- und Zersetzungsprodukten ins Abwasser gelangen.

Das erfindungsgemässe Verfahren wird durch das folgende Beispiel näher erläutert.

*Beispiel : Herstellung von N-(1'-Methoxycarbonyläthyl)-2,6-dimethylanilin:*

Eine Mischung von 132,5 (1,08 mol) 2-Chlorpropionsäuremethylester, 217,5 g (1,8 mol) 2,6-Dimethylanilin, 18,0 g (0,12 mol) Natriumjodid und 67,5 g (0,64 mol) Natriumcarbonat wird in einem mit Rührer, Thermometer und Kühler versehenen Dreihalskolben 14 h bei 123°C unter schwachem Vakuum gerührt, wobei gleichzeitig das bei der Umsetzung gebildete Wasser durch azeotrope Destillation entfernt wird. Nach beendigter Umsetzung wird das Reaktionsgemisch auf 80°C abgekühlt und mit 350 ml Wasser verrührt. Nach Abtrennung der wässerigen Phase wird die organische Phase destillativ aufgearbeitet. Es werden 80,0 g (0,66 mol) 2,6-Dimethylanilin und 217,0 g (93% d.Th. bezogen auf umgesetztes 2,6-Dimethylanilin) N-(1'-Methoxycarbonyläthyl)-2,6-dimethylanilin erhalten.

## Patentansprüche

1. Verfahren zur Herstellung von N-(1'-Alkoxycarbonyläthyl)-2,6-dialkylanilinen der Formel I:

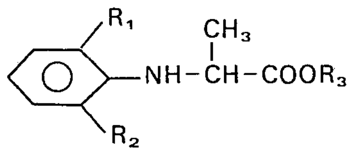

(I)

in welcher $R_1$ und $R_2$ je Methyl oder Äthyl bedeuten und $R_3$ eine Alkylgruppe mit 1 bis 4 Kohlenstoffatomen darstellt, durch Umsetzung eines 2,6-Dialkylanilins der Formel II:

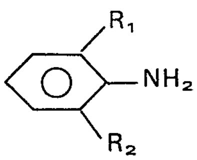

(II)

in welcher $R_1$ und $R_2$ die oben angegebene Bedeutung haben, mit einem 2-Halogenpropionsäureester der Formel III:

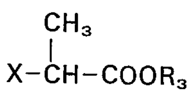

(III)

in welcher $R_3$ die oben angegebene Bedeutung hat und X für Chlor oder Brom steht, dadurch gekennzeichnet, dass man die Umsetzung des 2,6-Dialkylanilins der Formel II mit dem 2-Halogenpropionsäureester der Formel III bei 110 bis 130°C in Gegenwart eines Alkalijodids als Katalysator und eines Alkalicarbonats oder Alkalihydrogencarbonats als Säureakzeptor in überschüssigem 2,6-Dialkylanilin der Formel II als Lösungsmittel durchführt, während der Umsetzung das gebildete Wasser durch Destillation entfernt und die nach Zugabe von Wasser und Phasentrennung erhaltene organische Phase destillativ aufarbeitet.

2. Verfahren nach Anspruch 1, dadurch gekennzeichnet, dass man das 2,6-Dialkylanilin der Formel II in einer Menge von 1,5 bis 2,5 mol pro Mol 2-Halogenpropionsäureester der Formel III einsetzt.

3. Verfahren nach Anspruch 1, dadurch gekennzeichnet, dass man das 2,6-Dialkylanilin der Formel II in einer Menge von 1,6 bis 1,8 mol pro Mol 2-Halogenpropionsäureester der Formel III einsetzt.

4. Verfahren nach Anspruch 1, dadurch gekennzeichnet, dass man als Alkalijodid Natriumjodid oder Kaliumjodid verwendet.

5. Verfahren nach Anspruch 1, dadurch gekennzeichnet, dass man das Alkalijodid in einer Menge von 0,02 bis 0,3 mol pro Mol 2-Halogenpropionsäureester der Formel III einsetzt.

6. Verfahren nach Anspruch 1, dadurch gekennzeichnet, dass man das Alkalijodid in einer Menge von 0,03 bis 0,15 mol pro Mol 2-Halogenpropionsäureester der Formel III einsetzt.

7. Verfahren nach Anspruch 1, dadurch gekennzeichnet, dass man als säurebindendes Mittel Natrium-, Kalium-, Natriumhydrogen- oder Kaliumhydrogencarbonat verwendet.

8. Verfahren nach Anspruch 1, dadurch gekennzeichnet, dass man die als säurebindende Mittel verwendeten Alkalicarbonate und Alkalihydrogencarbonate in einer Menge von 1 bis 3 Eq. bezogen auf den zu bindenden Halogenwasserstoff einsetzt.

9. Verfahren nach Anspruch 1, dadurch gekennzeichnet, dass man die als säurebindende Mittel zu verwendenden Alkalicarbonate und Alkalihydrogencarbonate in einer Menge von 1,1 bis 1,3 Eq. bezogen auf den zu bindenden Halogenwasserstoff einsetzt.

10. Verfahren nach Anspruch 1, dadurch gekennzeichnet, dass man die Umsetzung eines 2,6-Dialkylanilins der Formel II mit einem 2-Halogenpropionsäureester der Formel III bei einer Temperatur von 120 bis 125°C durchführt.

11. Verfahren nach Anspruch 1, dadurch gekennzeichnet, dass man das bei der Umsetzung gebildete Wasser durch azeotrope Destillation abtrennt.

12. Verfahren nach Anspruch 1, dadurch gekennzeichnet, dass man pro Mol 2-Halogenpropionsäureester der Formel III 1,7 mol 2,6-Dialkylanilin der Formel II einsetzt und die Umsetzung bei 120 bis 125°C in Gegenwart von 0,03 bis 0,15 mol Natriumjodid pro Mol 2-Halogenpropionsäureester der Formel III und in Gegenwart von 0,55 bis 0,65 mol Natriumcarbonat pro Mol 2-Halogenpropionsäurrester der Formel III durchführt.

13. Verfahren nach Anspruch 1, dadurch gekennzeichnet, dass man als 2,6-Dialkylanilin der Formel II 2,6-Dimethylanilin verwendet.

14. Verfahren nach Anspruch 1, dadurch gekennzeichnet, dass man als 2-Halogenpropionsäureester der Formel III 2-Chlorpropionsäuremethylester verwendet.

## Revendications

1. Procédé de préparation de N-(1'-alcoxycarbonyléthyl)-2,6-dialkylanilines de formule I:

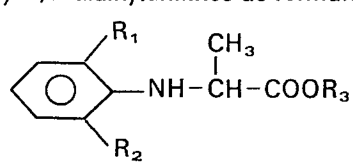

(I)

dans laquelle $R_1$ et $R_2$ représentent chacun un groupe méthyle ou éthyle et $R_2$ représente un

groupe alkyle en $C_1$-$C_4$, par réaction d'une 2,6-dialkylaniline de formule II:

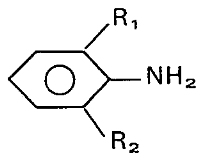 (II)

dans laquelle $R_1$ et $R_2$ ont les significations indiquées ci-dessus,
avec un ester d'acide 2-halogénopropionique de formule III:

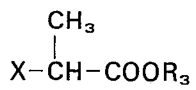 (III)

dans laquelle $R_3$ a la signification indiquée ci-dessus et X représente le chlore ou le brome,
caractérisé en ce que l'on effectue la réaction entre la 2,6-alkylaniline de formule II et l'ester d'acide 2-halogénopropionique de formule III à une température de 110 à 130°C en présence d'un iodure alcalin qui sert de catalyseur et d'un carbonate alcalin ou bicarbonate alcalin qui sert d'accepteur d' acide dans un excès de la 2,6-dialkylaniline de formule II qui sert de solvant, en éliminant l'eau formée dans la réaction par distillation, puis on traite par distillation la phase organique obtenue après addition d'eau et séparation des phases.

2. Procédé selon la revendication 1, caractérisé en ce que l'on met en œuvre la 2,6-dialkylaniline de formule II en quantité de 1,5 à 2,5 mol par mole de l'ester d'acide 2-halogénopropionique de formule III.

3. Procédé selon la revendication 1, caractérisé en ce que l'on met en œuvre la 2,6-dialkylaniline de formule II en quantité de 1,6 à 1,8 mol par mole de l'ester d'acide 2-halogénopropionique de formule III.

4. Procédé selon la revendication 1, caractérisé en ce que l'iodure alcalin utilisé est l'iodure de sodium ou l'iodure de potassium.

5. Procédé selon la revendication 1, caractérisé en ce que l'on utilise l'iodure alcalin en quantité de 0,02 à 0,3 mol par mole de l'ester d'acide 2-halogénopropionique de formule III.

6. Procédé selon la revendication 1, caractérisé en ce que l'on utilise l'iodure alcalin en quantité de 0,03 à 0,15 mol par mole de l'ester d'acide 2-halogénopropionique de formule III.

7. Procédé selon la revendication 1, caractérisé en ce que l'on utilise, en tant qu'agent fixant les acides, le carbonate de sodium, le carbonate de potassium, le bicarbonate de sodium ou le bicarbonate de potassium.

8. Procédé selon la revendication 1, caractérisé en ce que l'on met en œuvre les carbonates et bicarbonates alcalins utilisés comme agents fixant les acides en quantité de 1 à 3 Eq par rapport à l'halogénure d'hydrogène à fixer.

9. Procédé selon la revendication 1, caractérisé en ce que l'on met en œuvre les carbonates et bicarbonates alcalins à utiliser comme agents fixant les acides en quantité de 1,1 à 1,3 Eq par rapport à l'halogénure d'hydrogène à fixer.

10. Procédé selon la revendication 1, caractérisé en ce que l'on effectue la réaction entre une 2,6-dialkylaniline de formule II et un ester d'acide 2-halogénopropionique de formule III à une température de 120 à 125°C.

11. Procédé selon la revendication 1, caractérisé en ce que l'on sépare l'eau formée dans la réaction par distillation azéotropique.

12. Procédé selon la revendication 1, caractérisé en ce que l'on met en œuvre 1,7 mol de la 2,6-dialkylaniline de formule II par mole de l'ester d'acide 2-halogénopropionique de formule III et on effectue la réaction à une température de 120 à 125°C en présence de 0,03 à 0,15 mol d'iodure de sodium par mole de l'ester d'acide 2-halogénopropionique de formule III et en présence de 0,55 à 0,65 mol de carbonate de sodium par mole de l'ester d'acide 2-halogénopropionique de formule III.

13. Procédé selon la revendication 1, caractérisé en ce que la 2,6-dialkylaniline de formule II utilisée est la 2,6-diméthylaniline.

14. Procédé selon la revendication 1, caractérisé en ce que l'ester d'acide 2-halogénopropionique de formule III utilisé est le 2-chloropropionate de méthyle.

## Claims

1. A process for the preparation of N-(1'-alkoxycarbonylethyl)-2,6-dialkylanilines of the formula I:

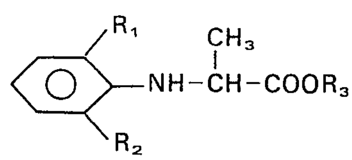 (I)

in which $R_1$ and $R_2$ are each methyl or ethyl and $R_3$ is an alkyl group having 1 to 4 carbon atoms, by reacting a 2,6-dialkylaniline of the formula II:

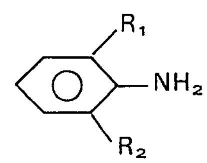 (II)

in which $R_1$ and $R_2$ are as defined above, with a 2-halogenopropionic acid ester of the formula III:

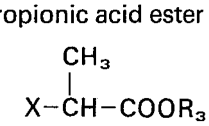 (III)

in which $R_3$ is as defined above and X is chlorine or bromine, which comprises carrying out the reaction of the 2,6-dialkylaniline of the formula II with the 2-halogenopropionic acid ester of the formula III at 110 bis 130°C, in the presence of an alkali metal iodide, as the catalyst, and of an alkali metal carbonate or alkali metal bicarbonate, as an acid acceptor, in excess 2,6-dialkylaniline of the formula II as the solvent, the water formed being removed by distillation during the reaction and the organic phase obtained after the addition of water

and separation of the phases being worked up by distillation.

2. A process according to claim 1, wherein the 2,6-dialkylaniline of the formula II is employed in an amount of 1.5 to 2.5 mol per mol of 2-halogenopropionic acid ester of the formula III.

3. A process according to claim 1, wherein the 2,6-dialkylaniline of the formula II is employed in an amount of 1.6 to 1.8 mol per mol of 2-halogenopropionic acid ester of the formula III.

4. A process according to claim 1, wherein the alkali metal iodide used is sodium iodide or potassium iodide.

5. A process according to claim 1, wherein the alkali metal iodide is employed in an amount of 0.02 to 0.3 mol per mol of 2-halogenopropionic acid ester of the formula III.

6. A process according to claim 1, wherein the alkali metal iodide is employed in an amount of 0.03 to 0.15 mol per mol of 2-halogenopropionic acid ester of the formula III.

7. A process according to claim 1, wherein the acid-binding agent used is sodium carbonate, potassium carbonate, sodium bicarbonate or potassium bicarbonate.

8. A process according to claim 1, wherein the alkali metal carbonate or alkali metal bicarbonate used as the acid-binding agent is employed in an amount of 1 to 3 Eq., based on the hydrogen halide to be bonded.

9. A process according to claim 1, wherein the alkali metal carbonate or alkali metal bicarbonate to be used as the acid-binding agent is employed in an amount of 1.1 to 1.3 Eq., based on the hydrogen halide to be bonded.

10. A process according to claim 1, wherein the reaction of a 2,6-dialkylaniline of the formula II with a 2-halogenopropionic acid ester of the formula III is carried out at a temperature of 120 to 125°C.

11. A process according to claim 1, wherein the water formed during the reaction is separated off by azeotropic distillation.

12. A process according to claim 1, wherein 1.7 mol of 2,6-dialkylaniline of the formula II are employed per mol of 2-halogenopropionic acid ester of the formula III and the reaction is carried out at 120 to 125°C in the presence of 0.03 to 0.15 mol of sodium iodide per mol of 2-halogenopropionic acid ester of the formula III, and in the presence of 0.55 to 0.65 mol of sodium carbonate per mol of 2-halogenopropionic acid ester of the formula III.

13. A process according to claim 1, wherein the 2,6-dialkylaniline of the formula II which is used is 2,6-dimethylaniline.

14. A process according to claim 1, wherein the 2-halogenopropionic acid ester of the formula III which is used is methyl 2-chloropropionate.